# EUROPEAN PATENT SPECIFICATION

(11) **EP 1 636 592 B1**
(45) Date of publication and mention of the grant of the patent: **19.12.2012**
(21) Application number: 04741839.7
(22) Date of filing: 18.06.2004
(51) Int. Cl.: C07K 16/18, A61K 31/00

(54) **USE OF PRION CONVERSION MODULATING AGENTS**
VERWENDUNG VON PRION-UMWANDLUNG MODULIERENDE MITTEL
UTILISATION D'AGENTS MODULATEURS DE LA CONVERSION DE PRIONS

(30) Priority: 19.06.2003 EP 03101795
(43) Date of publication of application: 22.03.2006
(73) Proprietor: Merck Serono SA, 1267 Coinsins, Vaud (CH)
(72) Inventor: SOTO-JARA, Claudio, Friendswood, TX 77546 (US); MAUNDRELL, Kinsey, CH-1224 Geneva (CH)
(74) Representative: Merck Serono SA - Geneva Intellectual Property
(86) International application number: PCT/EP2004/051170
(87) International publication number: WO 2004/111652

(56) References cited:
- WO-A-97/14437
- WO-A-99/15159
- WO-A-02/065133
- WO-A-03/005037
- US-A- 6 022 683
- US-A1- 2002 155 426
- US-A1- 2004 018 554
- US-B1- 6 462 171
- LINGAPPA VR ET AL: "Translocational pausing and the regulation of membrane protein biogenesis" MEMBRANE PROTEINS: STRUCTURE, FUNCTION AND EXPRESSION CONTROL KYUSHU UNIVERSITY PRESS, 7-1-146, HAKOZAKI, HIGASHI-KU, FUKUOKA 812, JAPAN; S. KARGER AG, P.O. BOX, ALLSCHWILERSTRASSE 10, CH-4009 BASEL, SWITZERLAND, 1997, pages 93-100, XP001183818 & INTERNATIONAL SYMPOSIUM ISSN: 3-8055-6465-1
- CLAVEY V ET AL: "INTERACTION ENTRE LE LDL-RECEPTEUR ET LES LIPOPROTEINES CONTENANT DE L'APO B INTERACTION BETWEEN THE LDL RECEPTOR AND THE LIPOPROTEINS CONTAINING APOB" ANNALES D'ENDOCRINOLOGIE, MASSON, PARIS, FR, vol. 52, no. 6, 1991, pages 459-463, XP001029770 ISSN: 0003-4266 cited in the application
- BAUMANN, MARC H. ET AL: "Apolipoprotein E includes a binding site which is recognized by several amyloidogenic polypeptides" BIOCHEMICAL JOURNAL, vol. 349, no. 1, 1 July 2000 (2000-07-01), pages 77-84, XP002262330 cited in the application
- DIEDRICH, JANE F. ET AL: "Neuropathological changes in scrapie and Alzheimer's disease are associated with increased expression of apolipoprotein E-- and cathepsin D in astrocytes" JOURNAL OF VIROLOGY, vol. 65, no. 9, September 1991 (1991-09), pages 4759-4768, XP000443989 cited in the application
- CHOE, LEILA H. ET AL: "Apolipoprotein E and other cerebrospinal fluid proteins differentiate ante mortem variant Creutzfeldt-Jakob disease from ante mortem sporadic Creutzfeldt-Jakob disease" ELECTROPHORESIS, vol. 23, no. 14, 14 July 2002 (2002-07-14), pages 2242-2246, XP002262331 cited in the application
- GOLAZ OLIVIER ET AL: "Phenotyping of apolipoprotein E using immobilized pH gradient gels for one-dimensional and two-dimensional separations" ELECTROPHORESIS, vol. 16, no. 7, 1995, pages 1184-1186, XP009021630 ISSN: 0173-0835 cited in the application
- LUCASSEN RALF ET AL: "In vitro amplification of protease-resistant prion protein requires free sulfhydryl groups" BIOCHEMISTRY;BIOCHEMISTRY APR 15 2003, vol. 42, no. 14, 15 April 2003 (2003-04-15), pages 4127-4135, XP002262517 cited in the application
- ENARI M FLECHSIG E WEISSMANN C: "Scrapie prion protein accumulation by scrapie-infected neuroblastoma cells abrogated by exposure to a prion protein antibody" PROCEEDINGS OF THE NATIONAL ACADEMY OF SCIENCES OF USA, NATIONAL ACADEMY OF SCIENCE. WASHINGTON, US, vol. 98, no. 16, 31 July 2001 (2001-07-31), pages 9295-9299, XP002959455 ISSN: 0027-8424 cited in the application

## Description

### Field of the invention

This invention relates to the use of apolipoprotein B or apolipoprotein E or fragments or mimetics thereof for diagnostic, detection, prognostic and identifying modulators of the prion protein replication. More specifically, the invention provides the use of modulators of apolipoprotein B or fragments thereof for modulating the prion protein replication which are implicated in the pathogenesis of transmissible spongiform encephalopathies and other prion diseases.

### Background of the invention

Creutzfeldt-Jakob disease (CJD) in humans and scrapie and bovine spongiform encephalopathy (BSE) in animals are some of the diseases that belong to the group of Transmissible Spongiform Encephalopathies (TSE), also known as prion diseases *(Prusiner, 1991).* These diseases are characterized by an extremely long incubation period, followed by a brief and invariably fatal clinical disease (*Roos et al., 1973*). To date no therapy is available.

Although these diseases are relatively rare in humans, the risk for the transmissibility of BSE to humans through the food chain has seized the attention of the public health authorities and the scientific community *(Soto at al., 2001).* Variant CJD (vCJD) is a new disease, which was first described in March 1996 *(Will et al., 1996).* In contrast to typical cases of sporadic CJD (sCJD), this variant form affects young patients (average age 27 years old) and has a relatively long duration of illness (median 14 months vs. 4.5 months in traditional CJD). A link between vCJD and BSE was first hypothesized because of the association of these two TSEs in place and time *(Bruce, 2000).* The most recent and powerful evidence comes from studies showing that the transmission characteristics of BSE and vCJD to mice are almost identical and strongly indicating that they are due to the same causative agent *(Bruce et al., 1997).* Morcover, transgenic mice carrying a human or a bovine gene have now been shown to be susceptible to BSE and vCJD *(Scott et al., 1999).* Furthermore, no other plausible hypothesis for the occurrence of vCJD has been proposed and intensive CJD surveillance in five European countries, with a low exposure to the BSE agent, has failed to identify any additional cases. In conclusion, the most likely cause of vCJD is exposure to the BSE agent, probably due to dietary contamination with affected bovine central nervous system tissue.

The nature of the transmissible agent has been matter ofpassionate controversy. Further research, has indicated that the TSE agent differs significantly from viruses and other conventional agents in that it seems not to contain nucleic acids *(Prusiner, 1998).* Additionally, the physicochemical procedures that inactivate most viruses, such as disrupting nucleic acids, have proved ineffective in decreasing the infectivity of the TSE pathogen. In contrast, the procedures that degrade protein have been found to inactivate the pathogen *(Prusiner, 1991).* Accordingly, the theory that proposes that the transmissible agent is neither a virus nor other previously known infectious agent, but rather an unconventional agent consisting only of a protein recently gained widespread acceptability *(Prusiner, 1998).* This new class of pathogen was called a "prion", short for "proteinaceous infectious particle". In TSE, prions are composed mainly of a misfolded protein named PrP^{Sc} (for scrapie PrP), which is a post-translationally modified version of a normal protein, termed PrP^{C} *(Cohen et al., 1998).* Chemical differences have not been detected to distinguish these two PrP isoforms and the conversion seems to involve a conformational change whereby the α-helical content of the normal protein diminishes and the amount of β-sheet increases *(Pan et al., 1993).* The structural changes are followed by alterations in the biochemical properties: PrP^{C} is soluble in non-denaturing detergents, PrP^{Sc} is insoluble; PrP^{C} is readily digested by proteases (also called protease sensitive prion protein) while PrP^{Sc} is partially resistant, resulting in the formation of a N-terminally truncated fragment known as PrPres (protease resistant prion protein) *(Cohen et al., 1998).*

The notion that endogenous PrP^{c} is involved in the development of infection is supported by experiments in which endogenous PrP gene was knocked out where the animals were both resistant to prion disease and unable to generate new infectious particles *(Bueler et al., 1993).* In addition, it is clear that during the time between the inoculation with the infectious protein and the appearance of the clinical symptoms, there is a dramatic increase in the amount of PrP^{Sc}.

These findings suggest that endogenous PrP^{C} is converted to PrP^{Sc} conformation by the action of an infectious form of the PrP molecule *(Soto et al., 2001).* Prion replication is hypothesized to occur when PrP^{Sc} in the infecting inoculum interacts specifically with host PrP^{C}, catalyzing its conversion to the pathogenic form of the protein. A physical association between the two isoforms during the infectious process is suggested by the primary sequence specificity in prion transmission *(Telling et al., 1994)* and by the reported *in vitro* generation of PrP^{Sc}-like molecules by mixing purified PrP^{C} with PrP^{Sc} *(Saborio et al., 2001).* However, the exact mechanism underlying the conversion is not known.

Investigations with chimeric transgenes showed that PrP^{C} and PrP^{Sc} are likely to interact within a central domain delimited by codons 96 and 169 *(Prusiner, 1996)* and synthetic PrP peptides spanning the region 109-141 proved to be able to bind to PrP^{C} and compete with PrP^{Sc} interaction *(Chabry et al., 1998).*

Based on data with transgenic animals, it has been proposed that additional brain factors present in the host are essential for prion propagation *(Telling et al., 1995).* It has been demonstrated previously that prion conversion does not occur under experimental conditions where purified PrP^{C} and PrP^{Sc} are mixed and incubated *(Saborio et al., 1999)* but that the conversion activity is recovered when the bulk of cellular proteins are added back to the sample *(Saborio et al., 1999).* This finding provides direct evidence that other factors present in the brain are essential to catalyse prion propagation.

The observation that cholesterol depletion decreases the formation of PrP^{Sc} whereas sphingolipid depletion increases PrP^{Sc} formation, suggested that "lipid rafts" (lipid domains in membranes that contain sphingolipids and cholesterol) may be the site of the PrP^{c} to PrP^{Sc} conversion reaction involving either a raft-associated protein or selected raft lipids *(Fantini et al., 2002).* However, the role of lipid rafts in prion infectivity is still unclear.

Several *in vitro* methods of detections of prions in a sample have been developed. The set of known detection methods, include PrP^{Sc} detection methods using specific ligand carriers selected from aminoglycans, fibronectin and Apolipoprotein A (*WO 02*/*065133);* methods using the monoclonal antibodies selected from Gö138, 3B5 and 12F10 (*Schulz et al., 2000*); methods based on the formation of a complex between PrP^{Sc} and Apolipoprotein H (WO 03/005037); or methods based on the PrP^{Sc} *in vitro* amplification called protein misfolding cyclic Amplification (PMCA) described in *Saborio et al., 2001* and *Lucassen et al., 2003.*

Apolipoprotein B is the major protein component of the two known atherogenic lipoproteins, Low Density Lipoproteins (LDL) and remnants of triglyceride-rich lipoproteins. The apolipropotein B concentration is considered to be a direct reflection of the number of atherogenic particles in the blood and has been proposed as a parameter for determining the risk of atherosclerosis. Biogenesis of Apolipoprotein B is believed to demonstrate translocational regulation (Lingappa et al., 1997)

Apolipoprotein E is a constituent of several plasma lipoprotein such as chylomicrons, very low-density lipoproteins (VLDL), and high-density lipoproteins (HDL) (*Lehninger et al., 1993*).

Apolipoprotein E has recently emerged as a major genetic risk factor for Alzheimer's disease, a neurodegenerative disorder (US 6,022,683) and upregulated in the cerebrospinal fluid of patients with variant CJD and Alzheimer's disease compared to patients with sporadic CJD (*Choe et al., 2002*). The Apolipoprotein E 4/4 phenotype is associated with increased risk of coronary heart diseases and Creutzfeld-Jakob disease (*Golaz et al., 1995*). Apolipoprotein E gene expression was found to be increased in astrocytes associated with the neuropatholigical lesions in a scrapie animal model (*Dietrich et al., 1991*). Apolipoprotein E was found to recognise a shared structural motif of amyloids and prion which, after induction, can accelerate the adoption of a beta-sheet conformation *(Baumann et al., 2000*).

Apolipoprotein B and B are ligands for the LDL receptor and are known for its prominent role in cholesterol transport and plasma lipoprotein metabolism via LDL receptor interactions Apolipoprotein B is notably able to form a complexe withe a LDL receptor. *(Segrest et al., 2001; Clavey et al., 1991).*

One approach to the treatment and prevention of prion diseases has been to develop agents for blocking the transformation of PrP^{c} into PrP^{Sc}. Some agents proposed were Congo red dye (US 5,276,059), nerve growth peptides (US 5,134,121), fragments of prion proteins (US 6,355,610), compounds that reduces Apolipoprotein E release in the brain tissue (US 2002/0155426), therapeutic agents that prevent Apolipoprotein E4 to interact with neuronal LDL receptor-related protein (WO 97/14437), compounds that increase Apolipoprotein E levels (WO 99/15159) and beta-sheet breaker peptides (US 5,948,763).

It would be desirable to develop new methods for identifying and inhibiting the prion conversion factor(s).

### Summary of the invention

It is an object of the invention to provide a use of peptides or proteins in an assay for the detection of PrP^{Sc} formation in a sample.

It is also an object of the invention to provide a use of peptides or proteins in a screening assay for identifying compounds that modulate the conversion of PrP^{c} into PrP^{Sc}.

It is further an object of the invention to provide a substance which is suitable for the treatment of, and/or prevention of, and/or delaying the progression of prion related disorders, notably, bovine spongiform encephalopathy (BSE) and Creutzfeld-Jacob Disease (CJD).

In a first aspect, the invention provides a use of Apolipoprotein B in an assay for the detection of PrP^{Sc} formation in a sample.

In a second aspect, the invention provides a use of Apolipoprotein B in a screening assay for identifying compounds that modulate the conversion of PrP^{c} into PrP^{Sc}.

In a third aspect, the invention provides a use of a modulator, preferably an inhibitor or an antagonist, of Apolipoprotein B for the preparation of a pharmaceutical composition for the treatment of a prion disease, notably, bovine spongiform encephalopathy (BSE) and a Creutzfeld-Jacob Disease (CJD).

In a fourth aspect, the invention provides a method for the diagnosis or detection of a prion disease within a subject suspected of suffering from such a disease which comprises (i) contacting a sample from said subject with Apolipoprotein B (ii) contacting the sample obtained from step (i) with PrP^{C} or PrP^{C} containing mixtures, such as brain homogenates, cell lysates, lipid rafts preparation; and (iii) determining the presence and/or amount of PrP^{Sc} in said sample.

In a fifth aspect, the invention provides a method of determining a marker that predisposes a subject to a prion disease, comprising (i) measuring a level of Apolipoprotein B and (ii) correlating said level of protein obtained in said measuring step with the occurrence of a prion disease.

In a sixth aspect, the invention provides a method for the detection of PrP^{Sc} formation within a sample, which assay comprises (i) contacting said sample with Apolipoprotein B ; (ii) contacting the sample obtained from step (i) with PrP^{C} or PrP^{C} containing mixtures, such as brain homogenates, cell lysates, lipid rafts preparation; and (iii) determining the presence and/or amount of PrP^{Sc} in said sample.

In a seventh aspect, the invention provides a method for identifying a compound which modulates, preferably inhibits or antagonizes, the transition of PrP^{C} into PrP^{Sc} comprising: (i) contacting said sample with Apolipoprotein B (a) in the presence of said compound and (b) in the absence of said compound; (ii) contacting the sample obtained from step (i) a and (i) b with PrP^{C} or PrP^{C} containing mixtures, such as brain homogenates, cell lysates, lipid rafts preparation; and (iii) determining the amount of PrP^{Sc} (a) in the presence of said compound and (b) in the absence of said compound.

In a eighth aspect, the invention provides an assay for the detection of PrP^{Sc} formation within a sample, which assay comprises (i) contacting said sample with Apolipoprotein B; (ii) contacting the sample obtained from step (i) with PrP^{C} or PrP^{C} containing mixtures, such as brain homogenates, cell lysates, lipid rafts preparation; and (iii) determining the presence and/or amount of PrP^{Sc} in said sample.

In a ninth aspect, the invention provides a screening assay for identifying a compound which modulates, preferably inhibits or antagonizes, the transition of PrP^{C} into PrP^{Sc} comprising: (i) contacting said sample with Apolipoprotein B; (a) in the presence of said compound and (b) in the absence of said compound; (ii) contacting the sample obtained from step (i) a and (i) b with PrP^{C} or PrP^{C} containing mixtures, such as brain homogenates, cell lysates, lipid rafts preparation; and (iii) determining the amount of PrP^{Sc} (a) in the presence of said compound and (b) in the absence of said compound.

### Detailed description of the invention

The following paragraphs provide definitions of various terms, and are intended to apply uniformly throughout the specification and claims unless an otherwise expressly set out definition provides a different definition.

The term "Gerstmann-Strassler-Scheinker Disease" abbreviated as "GSS" refers to a form of inherited human prion disease. The disease occurs from an autosomal dominant disorder. Family members who inherit the mutant gene succumb to GSS.

The term "prion" shall mean a transmissible particle known to cause a group of such transmissible conformational diseases (spongiform encephalopathies) in humans and animals. The term "prion" is a contraction of the words "protein" and "infection" and the particles are comprised largely if not exclusively of PrP^{Sc} molecules.

"Prions" are distinct from bacteria, viruses and viroids. Known prions include those which infect animals to cause scrapie, a transmissible, degenerative disease of the nervous system of sheep and goats as well as bovine spongiform encephalopathies (BSE) or mad cow disease and feline spongiform encephalopathies of cats. Four prion diseases known to affect humans are Kuru, Creutzfeldt-Jakob Disease (CJD), Gerstmann-Strassler-Scheinker Disease (GSS), and fatal familial insomnia (FFI) *(Prusinier, 1991).* As used herein prion includes all forms of prions causing all or any of these diseases or others in any animals used -- and in particular in humans and in domestic farm animals.

The term "lipid rafts" refers to small platforms, composed of sphingolipids and cholesterol in the outer exoplasmic layer, connected to Cholesterol in the inner cytoplasmic layer of the bilayer that have been reviewed recently *(Simons et al., 2000).* Lipid rafts can be isolated as they are insoluble in certain detergents such as triton X-100 at 4°C. Therefore, rafts can be purified as detergent-insoluble membranes (DIMs) or detergent-resistant membranes (DRMs) by ultracentrifugation on sucrose gradients. Rafts are enriched in GPI-anchored proteins, as well as proteins involved in signal transduction and intracellular trafficking. In neurons, lipid rafts act as platforms for the signal transduction initiated by several classes of neurotrophic factors *(Tsui-Pierchala et al., 2002).* Example for lipid rafts extraction is given in Example n° 2 §c.

The term "prion conversion factor" refers to a factor comprising proteins, lipids, enzymes or receptors that acts as a co-factor or auxiliary factor involved in the process of conversion of PrP^{C} into PrP^{Sc} and favors the onset and/or progression of the prion disease.

The terms "standardized prion preparation", "prion preparation" and the like are used interchangeably herein to describe a composition containing prions which composition is obtained for example from brain tissue of mammals substantially the same genetic material as relates to PrP proteins, e.g. brain tissue from a set of mammal which exhibit signs or prion disease or for example a composition which is obtained from chronically prion infected cells.

The terms "sensitive to infection", "sensitive to prion infection" and the like are use for a material from a mammal, including cells, that can be infected with an amount and type of prion which would be expected to cause prion disease or symptoms.

By analogy, the terms "resistant to infection", "resistant to prion infection" and the like are used for a material from a mammal, including cells which has the characteristic to be resistant when infected with an amount and type of prion which would be expected to cause prion disease or symptoms and remain uninfected even after several infective prion material inoculations.

The term "sample" refers to a biological extract from a mammal, including cell sample, body fluid, genetic material such as brain homogenate, cells, lipid rafts or purified peptides and proteins.

The term "incubation time" shall mean the time from inoculation of an animal with a prion until the time when the animal first develops detectable symptoms of disease resulting from infection, it also means the time from inoculation of material from a mammal, e.g. brain homogenate, cells, lipid rafts from cells, with prion until the time when the prion infection is detectable such as through the conversion of PrP^{C} into PrP^{Sc}. Several methods of detection of prion infection and PrP conversion are known by a person skilled in the art.

The terms "fraction" or "fragment" refer to any fragment of the polypeptidic chain of the compound itself, alone or in combination with related molecules or residues bound to it, for example residues of sugars or phosphates, or aggregates of the original polypeptide or peptide. Such molecules can result also from other modifications which do not normally alter primary sequence, for example *in vivo* or *in vitro* chemical derivativization of peptides (acetylation or carboxylation), those made by modifying the pattern of phosphorylation (introduction of phosphotyrosine, phosphoserine, or phosphothreonine residues) or glycosylation (by exposing the peptide to enzymes which affect glycosylation e.g., mammalian glycosylating or deglycosylating enzymes) of a peptide during its synthesis and processing or in further processing steps.

The terms "modulator" or "modulatory compound" refer to molecules that modify the functions and/or properties (such as receptor binding, lipid affinity, enzyme interaction, structural arrangement, synthesis, metabolism) of the natural protein. "Modulators" or "modulatory compounds" include "agonists" and antagonists". Modulators" include peptides, proteins or fragments thereof, peptidomimeties, organic compounds and antibodies.

The term "mimetic" refer to molecules that mimic the functions and/or properties (such as receptor binding, lipid affinity, enzyme interaction, structural arrangement, synthesis, metabolism) of a natural protein. These compounds have for example the property to either enhance a property of the natural protein (i.e. to lead to the same activity when the compound is added to the natural protein as obtained with an increase in concentration in the natural protein) or to exhibit the same property as a natural protein (i.e. to lead to the same activity when the compound replaces the natural protein). "Mimetics" include peptides, proteins or fragments thereof, peptidomimetics and organic compounds. Examples of Apolipoprotein E mimetics are described in US 20020128175 and WO 2004043403*.*

The terms "inhibitor" or "antagonist" refer to molecules that alter partially or impair the functions and/or properties (such as receptor binding, lipid affinity, enzyme interaction, structural arrangement, synthesis, secretion, metabolism) of the natural protein. "inhibitors" or "antagonists" include peptides, proteins or fragments thereof, peptidomimetics, organic compounds and antibodies. Examples of Apolipoprotein B antibodies, are described in *Choi et al., 1997* and in *Wang et al., 2000.* Examples of Apolipoprotein antagonists can be antagonists that alter or impair the role of Apolipoproteins B in the cholesterol transport pathway. Examples of compounds that alter Apolipoprotein B secretion or synthesis are described in US 6,369,075*,* US 6,197,972*,* WO 03/002533 and WO 03/045921*.* Other "modulators" or "antagonists" can be modulators of the LDL receptor, preferably LDL-receptor antagonists such as anti-LDL receptor antibodies. Examples of monoclonal antibodies to the LDL receptor are given in WO 01/68710*.*

The term "protein misfolding cyclic amplification assay" or "PMCA assay" is an assay that for the diagnosis or detection of conformational diseases which comprises a cyclic amplification system to increase the levels of the pathogenic conformer such as described for example in WO 02/04954*.*

The term "marker" for a disease refers to a biological parameter or value including a genetic character, inherited protein mutation(s), blood level of a protein or an enzyme that is different from the average value in a heterogeneous population of individuals and whose occurrence correlates with the occurrence of said disease with a statistical significance. A "marker" for a disease or condition is typically defined as a certain cutoff level of a said biological variable. A "marker" provides basis for determining the risk (probability of occurrence) of a disease in a subject.

The term "complex" includes the formation of an entity by the interaction of several molecules, several proteins, several peptides together or with a receptor. These interactions may be reversible and/or transient. These interactions may induce changes in the properties of the interacting molecules, proteins, peptides or receptors.

By "effective amount", it is meant a concentration of peptide(s) that is capable of slowing down or inhibiting the formation of PrP^{Sc} deposits, or of dissolving preformed deposits. Such concentrations can be routinely determined by those of skill in the art. It will also be appreciated by those of skill in the art that the dosage may be dependent on the stability of the administered peptide. A less stable peptide may require administration in multiple doses.

The preparation of antibodies is known by the person skilled in the art. It is referred by "antibody" to a monoclonal antibody, chimeric antibody, humanized antibody, anti-anti-Id antibody or fragment thereof which specifically recognises and binds to Apo B or Apo E and fragments thereof. For example, monoclonal antibodies are obtained though the generation of hybridoma cells lines producing monoclonal antibodies capable of specifically recognising and binding Apo B and/or fragments thereof. More specifically, these monoclonal antibodies are capable of specifically recognising and binding Apo B. A monoclonal antibody can be prepared in a conventional manner, e.g. by growing a cloned hybridoma comprising a spleen cell from a mammal immunized with hApo B and a homogenic or heterogenic lymphoid cell in liquid medium or mammalian abdomen to allow the hybridoma to produce and accumulate the monoclonal antibody. Preferably, the antibody specifically recognises and binds to Apo B-LDL recognizing fragments.

The present invention provides compounds capable of controlling, including increasing and/or inhibiting, the conversion of PrP^{C} into PrP^{Sc} in prion diseases.

The activity of the compounds of the invention in controlling the conversion of PrP^{C} into PrP^{Sc} in prion diseases can be detected using, for example, an *in vitro* assay, such as that described by *Saborio et al., 2001* which measures the ability of compounds of the invention to modulate the conversion of PrP^{C} into PrP^{Sc}. Results are reported in the Examples.

In one embodiment, the invention provides a use of Apolipoprotein B in an assay for the detection of PrP^{Sc} formation in a sample.

In one further embodiment of the invention, Apolipoprotein B used in an assay for the detection of PrP^{Sc} formation in a sample binds and/or forms a complex with the LDL receptor.

In another embodiment, the invention provides a use of Apolipoprotein B in a screening assay for the identifying compounds that modulate the conversion of PrP^{c} into PrP^{Sc}.

In another further embodiment of the invention, Apolipoprotein B , is used in a screening assay for the identifying compounds that modulate the conversion of PrP^{c} into PrP^{Sc} binds and/or forms a complex with the LDL receptor.

In a further embodiment of the invention, the assay is a Protein Misfolding Cyclic (PMCA) assay.

In a preferred embodiment of the invention, the Protein Misfolding Cyclic (PMCA) assay uses normal brain homogenate as a source of normal PrP^{c} and prion conversion factor.

In a preferred embodiment of the invention, the Protein Misfolding Cyclic (PMCA) assay uses cell lysates or lipid rafts extracted from prion infection sensitive neuroblasma cells, such as line N2a, described in Example 2, and equivalent, as a source of normal PrP^{c} and prion conversion factor. Lipid raft fractions can also be purified directly from the brain to serve as a source of substrate for PMCA.

In a preferred embodiment, the invention provides a use of Apolipoprotein Bin an assays for the detection of PrP^{C} in a sample, wherein the assay is a Protein Misfolding Cyclic Amplification (PMCA) assay using lipid rafts from infection sensitive neuroblasma cell line N2a as a source of normal PrP^{C} and substrate.

In another embodiment, the invention provides a use of a modulator, preferably an inhibitor or an antagonist, of Apolipoprotein B for the preparation of a pharmaceutical composition for the treatment of a prion disease, notably, bovine spongiform encephalopathy (BSE) and Creutzfeld-Jacob Disease (CJD). The modulator modifies for example the functions and/or properties of Apolipoliprotein B or of a fragment thereof.

In a further embodiment of the invention, the modulator, preferably an inhibitor or an antagonist, of Apolipoprotein B which modifies, preferably inhibits the binding and/or the formation of a complex between Apolipoprotein B and the LDL receptor. An example of such modulator can be a LDL receptor modulator, such as a LDL-receptor antagonist such as an anti-LDL receptor antibody.

In a preferred embodiment of the invention, the modulator is an antagonist to Apolipoprotein B.

In a further preferred embodiment of the invention, the modulator is an antibody raised against Apolipoprotein B.

In another preferred embodiment of the invention, the modulator is an antibody raised against Apolipoprotein B.

In an embodiment of the invention, the invention provides a method for the diagnosis or detection of a prion disease within a subject suspected of suffering from such a disease which comprises (i) contacting a sample from said subject with Apolipoprotein B (ii) contacting the sample obtained from step (i) with PrP^{C} or PrP^{C} containing mixtures, such as brain homogenates, cell lysates, lipid rafts preparation; and (iii) determining the presence and/or amount of PrP^{Sc} in said sample. The sample from the subject includes a biological extract from a mammal such as cell sample, genetic material, body fluid, brain homogenate, cells and lipid rafts.

In another embodiment of the invention, the invention provides a method of determining a marker that predisposes a subject to a prion disease, comprising (i) measuring the level of Apolipoprotein B in said sample; (ii) contacting the sample obtained from step (i) with PrP^{C} or PrP^{C} containing mixtures, such as brain homogenates, cell lysates, lipid rafts preparation; and (iii) correlating said level of protein obtained in said measuring step with the occurrence of a prion disease. The maker includes a biological parameter or value such as a genetic character, inherited protein mutation(s), blood level of a protein or an enzyme.

In another embodiment of the invention, the invention provides a method for the detection of PrP^{Sc} formation within a sample, which assay comprises (i) contacting said sample with Apolipoprotein B (ii) contacting the sample obtained from step (i) with PrP^{C} or PrP^{C} containing mixtures, such as brain homogenates, cell lysates, lipid rafts preparation; and (iii) determining the presence and/or amount of PrP^{Sc} in said sample. The sample can be a biological preparation for which the presence of prion is to be detected for quality control reasons and/or a sample extracted from a subject that is suspected of suffering of such a disease, including a biological extract from a mammal such as cell sample, genetic material, body fluid, brain homogenate, cells and lipid rafts.

In another embodiment of the invention, the invention provides a method for identifying, in a sample, a compound which modulates, preferably inhibits or antagonizes, the transition of PrP^{C} into PrP^{Sc} comprising: (i) contacting said sample with Apolipoprotein B (ii) contacting the sample obtained from step (i) (a) in the presence of said compound and (b) in the absence of said compound; (iii) contacting the sample obtained from step (i) a and (i) b, with PrP^{C} or PrP^{C} containing mixtures, such as brain homogenates, cell lysates, lipid rafts preparation; and (iv) determining the amount of PrP^{Sc} (a) in the presence of said compound and (b) in the absence of said compound. The modulator, includes antibodies, inhibitors of Apolipoproteins B binding, including binding to the LDL receptor, and/or secretion and/or synthesis.

Still another embodiment of the present invention, is a method for treating or preventing a prion disease such as bovine spongiform encephalopathy (BSE) and Creutzfeld-Jacob Disease (CJD), wherein the method comprises administering an effective dose of the above-mentioned modulator of a peptide or a protein, wherein the peptide or the protein is selected from Apolipoprotein B to a subject in the need thereof, wherein the subject can be human or animal.

In a preferred method of use of the modulators, preferably inhibitors, administration of the modulators is by injection or infusion, at periodic intervals. The administration of a compound of the invention may begin before any symptoms are detected in the patient, and should continue thereafter.

The above-mentioned modulatory compounds of the present invention may be administered by any means that achieves the intended purpose. For example, administration may be by a number of different routes including, but not limited to subcutaneous, intravenous, intradermal, intramuscular, intraperitoneal, intra-cerebral, intrathecal, intranasal, oral, rectal, transdermal, intranasal or buccal. Preferably the compounds of the invention are administered by subcutaneous, intramuscular or intravenous injection or infusion.

Parenteral administration can be by bolus injection or by gradual perfusion over time. A typical regimen for preventing, suppressing, or treating prion related disorders, comprises either (1) administration of an effective amount in one or two doses of a high concentration of modulatory in the range of 0.5 to 10 mg of peptide, more preferably 0.5 to 10 mg of peptide, or (2) administration of an effective amount of the peptide in multiple doses of lower concentrations of modulatory compounds in the range of 10-1000 µg, more preferably 50-500 µg over a period of time up to and including several months to several years. It is understood that the dosage administered will be dependent upon the age, sex, health, and weight of the recipient, concurrent treatment, if any, frequency of treatment, and the nature of the effect desired. The total dose required for each treatment may be administered by multiple doses or in a single dose.

Preparations for parenteral administration include sterile aqueous or non-aqueous solutions, suspensions, and emulsions, which may contain auxiliary agents or excipients which are known in the art. Suitable formulations for parenteral administration include aqueous solutions of the active compounds in water-soluble form, for example, water-soluble salts. In addition, suspension of the active compound as appropriate oily injections suspensions may be administered.

In another embodiment of the invention is provided an assay for the detection of the formation of PrP^{Sc} within a sample, which assay comprises (i) contacting said sample with Apolipoprotein B (iii) contacting the sample obtained from step (iii) contacting the sample obtained from step (ii) with PrP^{C} or PrP^{C} containing mixtures, such as brain homogenates, cell lysates, lipid rafts preparation; and (iv) determining the presence and/or amount of PrP^{Sc} in said sample. The sample can be a biological preparation for which the presence of prion is to be detected for quality control reasons and/or a sample extracted from a subject that is suspected of suffering of such a disease, including a biological extract from a mammal such as cell sample, genetic material, body fluid, including blood, serum, plasma, brain homogenate, cells and lipid rafts.

In another embodiment of the invention, is provided a screening assay for identifying a compound which modulates, preferably inhibits or antagonizes, the transition of PrP^{C} into PrP^{Sc} comprising: (i) contacting said sample with Apolipoprotein B (a) in the presence of said compound and (b) in the absence of said modulatory compound; (ii) contacting the sample obtained from step (i) a and (i) b with PrP^{C} or PrP^{C} containing mixtures, such as brain homogenates, cell lysates, lipid rafts preparation; and (iii) determining the amount of PrP^{Sc} (a) in the presence of said compound and (b) in the absence of said modulatory compound. The modulator, includes antibodies, inhibitors of Apolipoproteins B and/or secretion and/or synthesis.

In a preferred embodiment, the prion disease is bovine spongiform encephalopathy (BSE).

In a preferred embodiment, the prion disease is sporadic, variant, familial or iatrogenic Creutzfeld-Jacob Disease (CJD).

The present invention has been described with reference to the specific embodiments, but the content of the description comprises all modifications and substitutions, which can be brought by a person skilled in the art without extending beyond the meaning and purpose of the claims.

The invention will now be described by means of the following Examples, which should not be construed as in any way limiting the present invention. The Examples will refer to the Figures specified here below.

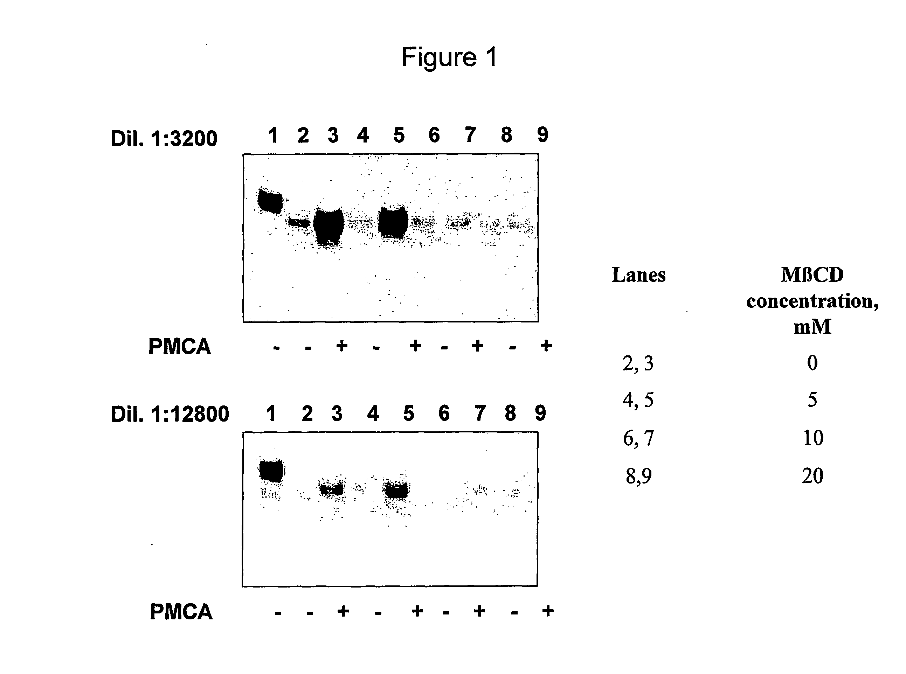

**Brief description of the drawings:** **Figure 1** **shows *in vitro* prion replication on Hamster brain homogenate by PMCA assay in presence and absence of a cholesterol-depleting agent (Example 1§b).** Samples contain 5% normal hamster brain homogenate incubated for 30 min at 4°C

with 0, 5, 10 or 20 mM (final concentration) of methyl-β-cyclodextrin (MßCD). Aliquots of scrapie brain homogenate are added to reach a 3200- (top panel) and 12800-fold (bottom panel) dilution. Half of the samples are frozen immediately as a control without amplification (PMCA "-") and the other half are subjected to 10 cycles of PMCA (PMCA "+"). Prion replication is evaluated by Western Blot after treatment of the samples with PK (100µg/ml for 60 min). The first lanes in each blot corresponds to the normal brain homogenate not treated with PK.

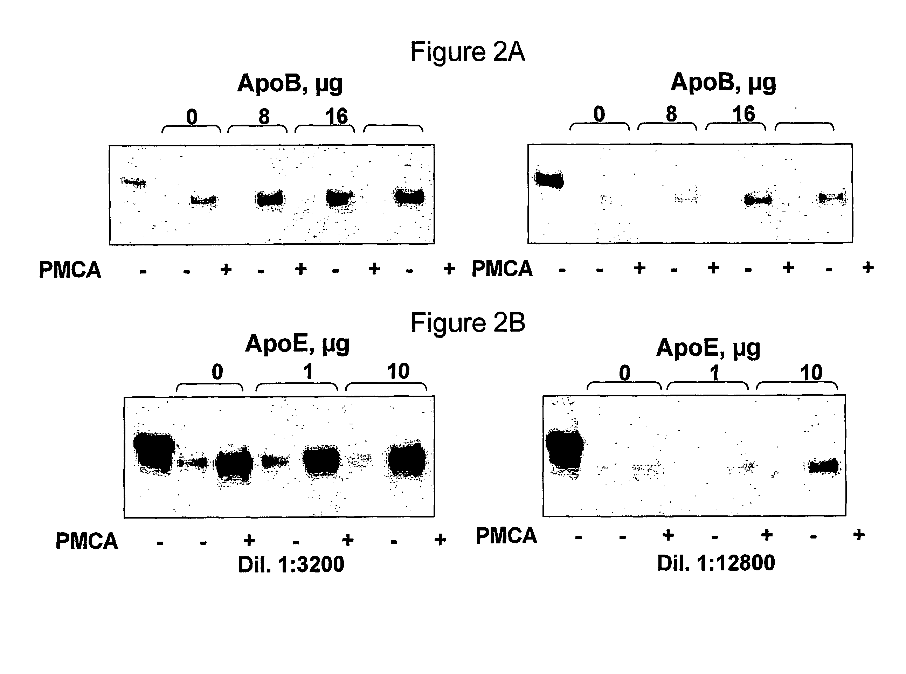

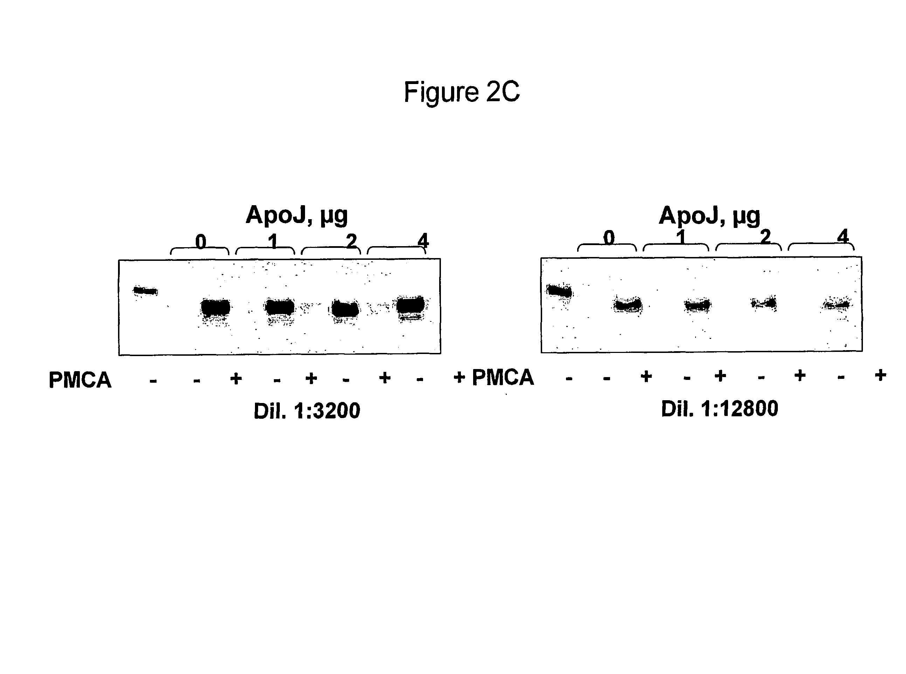

**Figure 2** **shows the effect of Apolipoproteins B, E and J *in vitro* prion replication on Hamster brain homogenate by PMCA assay (Example 1§c).** Samples containing 5% normal hamster brain homogenate are incubated with different quantities of human Apolipoprotein B (2A), human Apolipoprotein E (2B) or murine Apolipoprotein J (2C) for 30 min at 4°C. Aliquots of scrapie brain homogenate are added to reach a 3200- (left panel) or 12800-fold (right panel) dilution. Half of the samples are frozen immediately as a control without amplification (PMCA "-") and the other half are subjected to 10 cycles of PMCA (PMCA "+"). Prion replication is evaluated by western blot after treatment of the samples with PK (100µg/ml for 60 min). The first lanes in each blot corresponds to the normal brain homogenate not treated with PK.

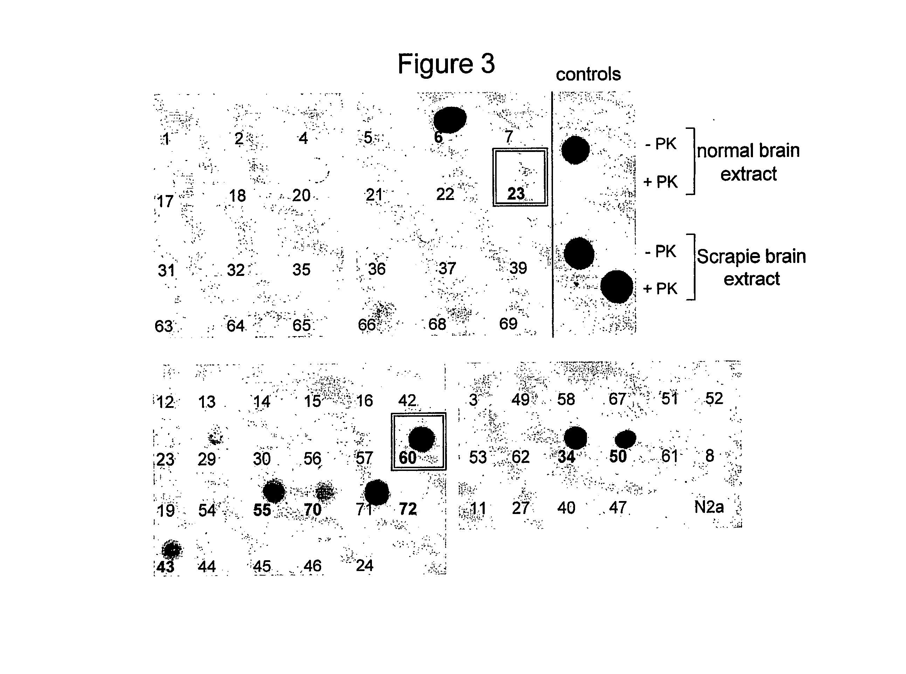

**Figure 3** **reports differential sensitivity of N2a sub-clones to infection by Scrapie revealed by exposure to anti-PrP 6H4 mabs (Example n° 2 §b).** Proteinase K (PK) exposure shows were the PrP^{Sc} isoform (Proteinase K resistant) is present. The two sub-clones highlighted #23 and #60 are chosen respectively as representatives of prion infection resistant and sensitive cells. 'N2a' shows uninfected N2a cells processed in parallel. Controls for blotting and PK digestion show 1 µl normal or scrapie brain extract diluted in 80µl lysis buffer and processed in parallel.

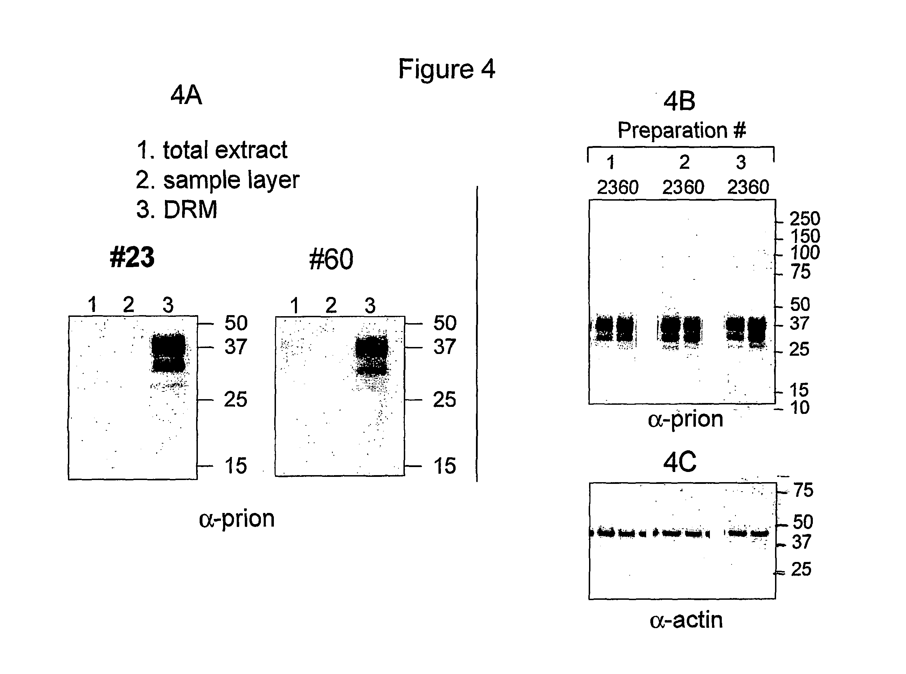

**Figure 4** **shows the characterization of PrP in lipid rafts from sub-clones prion infection resistant (#23) and sensitive (#60) N2a (Example 2 §c).** **Figure 4A** shows PrP quantification by Western blotting in lipid rafts which are extracted from prion infection resistant (#23) and sensitive (#60) cells. The distribution of PrP in the total extract (25µg loaded) **(1),** the sucrose sample layer after centrifigation (25µg loaded) **(2)** and the bouyant lipid raft fraction (4µg loaded) **(3)** are presented. **Figure 4B** shows PrP content and glycosylation pattern of the two sub-clones #23 and #60 by Western blotting with anti-PrP. Three independent preparations of lipid rafts prion infection resistant (#23) and sensitive (#60) cells were analysed. Equal amounts (4µg) of rafts proteins were analysed in each case. **Figure 4C** shows the same membrane after stripping and re-probing with anti-actin which confirms the similarity in protein loading.

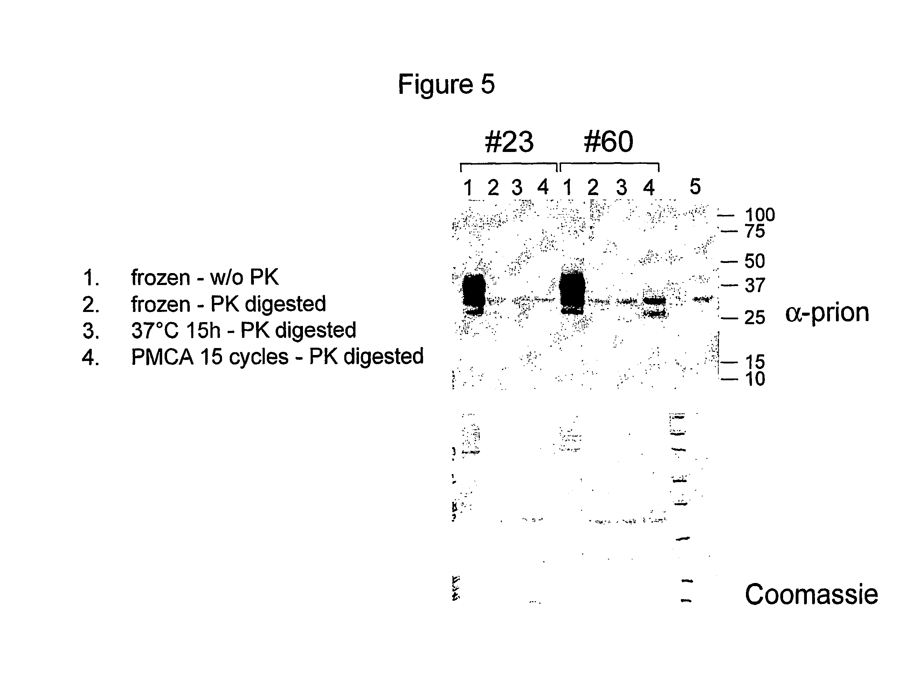

**Figure 5** **presents the *in vitro* conversion activity of lipid rafts from sub-clones prion infection resistant (#23) and sensitive (#60) N2a using PMCA (Example 2 §d).** *Upper panel:* Lipid rafts are isolated from prion infection resistant (#23) and sensitive (#60) cells. Preparations are mixed in a ratio 100:1 with 10% RML brain homogenate and aliquots are frozen immediately, incubated for 15h at 37 °C or subjected to 15 cycles of PMCA. Lanes 1: initial mixture without PK digestion; lanes 2: initial mixture digested 10ug/ml PK 1hr 37 C; lanes 3: mixture incubated 37 °C PK digested as in lane 2; lanes 4: 15 cycles of PMCA followed by PK digestion as in lane 2. Lane 5 shows the migration and cross-reactivity with anti-PrP of PK alone. *Lower* panel: Following western blotting the membrane is stained with Coomasie blue to confirm that digestion with PK was complete.

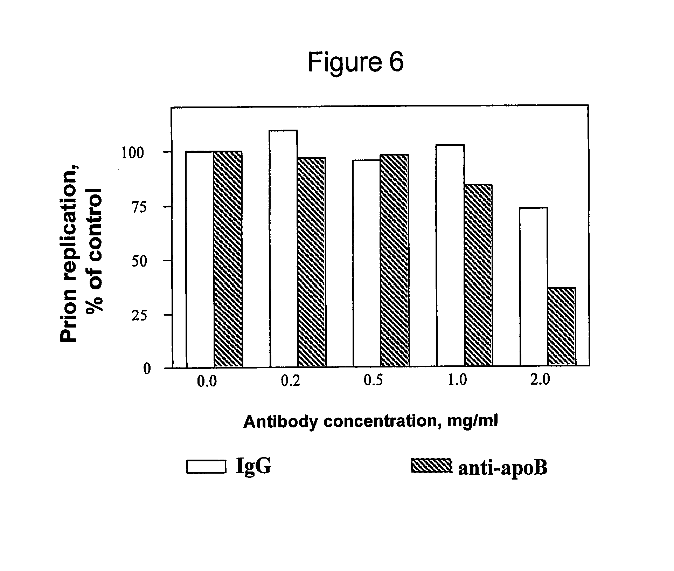

**Figure 6** **shows the inhibitory effect on Prion replication in prion infection sensitive cells induced by Anti-hApoB polyclonal antibody (Example 2 §e).**
   Chronically infected #60 sensitive cells were cultured in 24 well culture dishes in the presence of increasing amounts (0-2mg/ml) of a goat polyclonal antibody against human ApoB (Chemicon) or against a corresponding series of naive goat IgG. The level of PrP replication was determined by quantitative dot blotting and expressed as chemiluminescent intensity/mg protein. In the graph, for each antibody concentration the chemiluminescent intensity is expressed as a percentage of the value obtained without the antibody. Higher concentrations of anti-hApoB antibody have an inhibitory effect on PrP replication.

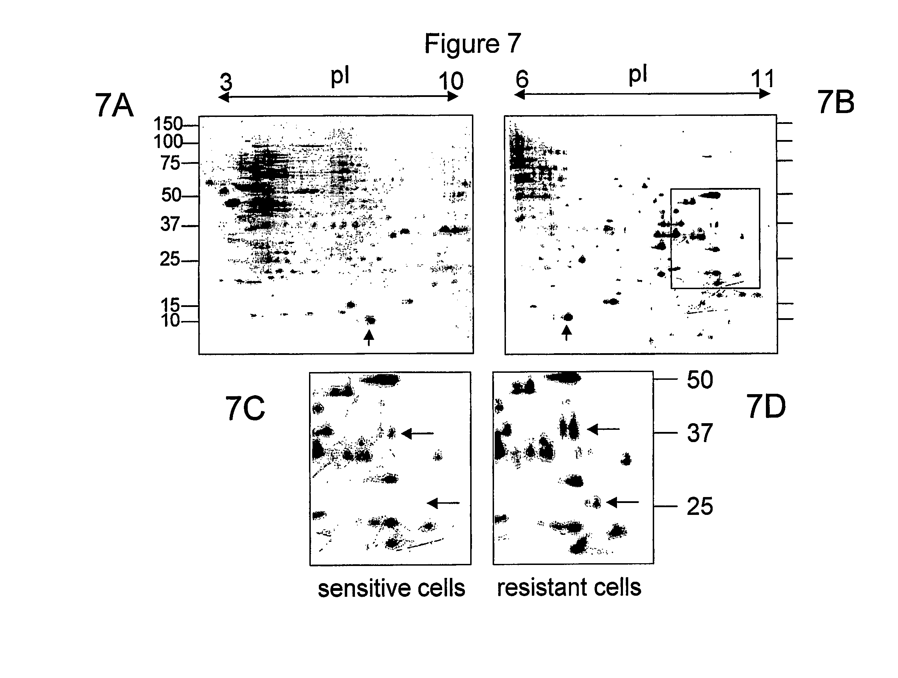

**Figure 7** **shows 2D separations of lipid raft proteins from N2a cells (Example 3).** Lipid rafts are isolated from prion infection sensitive cells (#60) and 2 aliquots of 25 µg are precipitated with acetone and processed for 2D analysis min the 1st dimension spanning pH ranges 3-10 (7A) or 6-11 (7B). After SDS-PAGE separation in the second dimension, gels are stained using the silver express kit (Invitrogen). Arrow indicates the same protein on both gels (7A and 7B). Proteins within the rectangle shown in B are compared between lipid raft from the prion infection sensitive sub-clone #60, (C) and resistant subclone #23, (D). Arrows indicate proteins which are more abundant in resistant cells.

### Abbreviations:

Apo B (Apolipoprotein B; Apo E (apolipoprotein E); Apo J (Apolipoprotein J); BCA (Bicinchoninic Acid); CHAPS (3-((3-cholamidopropyl)dimethylammonio)-1-propanesulfonate); CNS (central nervous system); BSE (bovine spongiform encephalopathy); CJD (Creutzfeldt-Jakob Disease); DiI (1,1-dioctadecyl-3,3,3 ,3 - tetramethylindocarbocyanine perchlorate); DIM (Detergent-Insoluble Membrane); DMEM (Dulbeceo's Modified Eagle Medium); DRM (Detergent Resistant Membrane); DTT (1,4-Dithio-D,L-threitol); IPG (Immobilized PH Gradient); IEF (Isoclectric Focusing); FCS (Fetal Calf Serum); FFI (Fatal Familial Insomnia); GSS (Gerstmann-Strassler-Scheinker Disease); hr (hour); HRP (Horseradish Peroxidase); kDa (KiloDalton); LDL (Low Density Lipoprotein); µg (microgram); µl (microliter); min (minute); MBCD (methyl-β-cyclodextrin); mM (millimolar); MS (mass spectrometry); PBS (Phosphate Buffered Sulfate); PK (proteinase K); PMCA (Protein Misfolding Cyclic Amplification); PMSF (Phenylmethanesulfonyl Fluoride); PrP (prion protein); PrP^{C} (normal, non-pathogenic conformer of PrP); PrP^{Sc} (pathogenic or "scrapie" isoform of PrP which is also the marker for prion diseases); PVDF (polyvinylidene difluoride); RPM (Rotation per minute); RML (Rocky Mountain Laboratory); RT-PCR (reverse transcriptase polymerase chain reaction); SDS (Sodium Dodecyl Sulfate); V (Volt); Vol. (volume).

### EXAMPLES

The invention will be illustrated by means of the following examples which are not to be construed as limiting the scope of the invention.

The following examples illustrate preferred compounds and methods for determining their biological activities.

PrP scrapie used as infection innoculum is RML (Rocky Mountain Laboratory) strain. Anti-PrP 6H4 monoclonal antibodies were purchased from Prionics.

Proteinase K was obtained from Boerhinger Ingelheim and Methyl.β.cyclodextrin from Sigma.

Purified and delipidated human Apolipoprotein B (Apo B) and Apolipoprotein E (Apo E) were obtained from Chemicon.

Anti-apo B and anti-apo E are goat polyclonal antibodies against human Apo B and human Apo E, respectively obtained from Chemicon and dialysed against PBS to eliminate sodium azide.

Total goat IgG was purchased from Pierce and dialyzed against PBS.

Mouse neuroblasma N2a cell line was obtained from ATCC.

Murine Apo J (Apo J) was obtained in-house as described in PCT/EP2004/05037.

DiI labeled LDL was obtained from Molecular Probes (L-3482).

**EXAMPLE 1:** *In vitro* prion replication in brain homogenate through PMCA assay: The influence of cholesterol and some of the apolipoproproteins on prion replication in vitro is analysed through a Protein Misfolding Cyclic Amplification assay (PMCA) (*Saborio et al., 2001*) where hamster brain homogenate is used as a source of PrP^{C} and conversion factors as follows.

### a) Brain preparation:

Brains from healthy Syrian golden hamsters healthy or infected with the adapted scrapie strain 263 K are obtained after decapitation and immediately frozen in dry ice and kept at -80° until used. Brains are homogenized in PBS containing protease inhibitors (Complete™ cocktail from Boehringer Mannheim) at a 1x final concentration. Detergents (0.5% Triton X-100, 0.05% SDS, final concentrations) are added and samples clarified with low speed centrifugation (10000 g) for 1 min, using an Eppendorf centrifuge (model 5415).

Dilutions (3200-fold and 12800-fold) of the scrapie brain homogenate are added directly to the healthy brain homogenate to trigger prion replication. 60µl of these mixtures are frozen immediately and another 60µl are incubated at 37°C with agitation. Each hour a cycle of sonication (5 pulses of 1sec each) is done using a microsonicator (Bandelin Electronic, model Sonopuls) with the probe immersed in the sample and the power setting fixed at 40%. These cycles are repeated 10 times.

### b) PMCA signal in presence and absence of a cholesterol-depleting agent:

Under these conditions a dramatic increase in the amount of PrP^{Sc} signal is observed after 10 cycles of PMCA (Figure 1, lanes 2 and 3). When normal brain homogenate is treated during 30 min with 10 and 20mM (but not 5 mM) methyl-β-cyclodextrin (MβCD) a complete inhibition of prion replication is observed (Figure 1, lanes 6-9) as obtained in mouse models in cell cultures and in vitro, indicating that cholesterol depletion has a detrimental effect on prion replication *(Taraboulos et al., 1995).*

### c) PMCA signal in presence of apolipoproteins

Purified delipidated human ApoB (Figure 2A) and human Apo E (Figure 2B) are respectively added to the PMCA preparation without cyclodextrin at different concentrations (8 and 16 µg for hApo B) and (1 and 10 µg for hApo E). Samples are incubated for 30 min at 4°C and thereafter half of each sample is frozen and the other half subjected to PMCA cycles.

An increase in prion replication in vitro is observed at both 3200-fold and 12800-fold dilutions of scrapie brain homogenate for both Apolipoprotein B and Apolipoprotein E.

In contrast, addition of Apolipoprotein J (at concentrations of 1, 2 and 4 µg), an Apolipoprotein component of HDL, has no effect on PMCA signal (Figure 2C).

These data show the effect of Apolipoprotein B and E implicated in the prion conversion.

### EXAMPLE 2: In vitro prion replication in lipid rafts from prion infection sensitive cells by PMCA:

The mouse neuroblastoma cell line N2a is used for their capability to be infected with PrPSc. Baron et al., 2002 and Enari et al., 2001 have shown that prion infection sensitive and prion infection resistant N2a sub-lines exist Lipid rafts from the prion infection sensitive line are isolated and used as a substrate for PMCA assay. The effect of an apolipoprotein B antagonist on prion conversion is studied through the ability of apolipoprotein B antagonist to inhibit the prion replication ability of prion infection sensitive N2a cell lines.

### a) N2a cell preparation:

Sub-clones of the parental mouse neuroblastoma N2a cell line are derived from single cells by limit dilution. A growing culture (Dulbecco's Modified Eagles Medium (DMEM Gibco # 41966-029), containing 10% fetal calf serum (FCS) and supplemented with 2mM, L-glutamate and standard antibiotics (penicillin and streptomycin)) is diluted to a density of 5 cells/ml and 100µl is transferred to individual wells of a 96 well plate and cultured for 1 week.

The individual cultures arc examined microscopically to determine those wells which contained a single focus of growing cells. The single cell derived cultures are then transferred to 24 well plates and serially passaged every 3-4 days at 1:15 dilution to maintain stocks. A total of 63 cultures are isolated and all tested for sensitivity to infection by the RML strain of PrP^{Sc}. To do this, 4µl of a 10% late stage infected brain extract is added per well of newly passaged cells, and the cultures are left for a further 4 days to reach 100 % confluence. Cells were serially passaged thereafter in the absence of PrP^{Sc}. Tests showed that all trace of the initial innoculum disappeared by passage 4.

At this and later passages individual cultures are tested for the presence of PrP^{Sc}.

### b) Prion infection resistant cell isolation by Cell culture dot blotting:

The presence of PrP^{Sc} in the 63 individual cell cultures is tested by cell culture dot blotting procedure in which lysis and proteinase K (PK) digestion are carried out directly in the culture dish. PK resistant PrP^{Sc} is detected by dot blotting to PVDF membranes and exposure to anti-PrP antibody as follows:

Infected cells are grown for 3-4 days in 24-well plates and washed once with PBS. 40µl DNasel (1000U/ml in H20) is added to each well at room temperature for 5min, followed by 40µl proteinase K solution (20µg/ml in 100mMTris/HCl pH 7.4, 300mM NaCl, 1% Triton-X100, 1% sodium deoxycholate). Plates are incubated at 37°C for 1hr with gentle agitation. Proteinase K digestion is stopped by addition of 2µl of a solution containing 80µg/ml PMSF, 10mmTris-HCl pH 8.0 and 1mg/ml bromophenol blue. 20µl aliquots are spotted onto PVDF membranes equilibrated with a degasses solution containing 192mM Glycine, 25mM Tris, 20% methanol. Membranes are then transferred to 3M guanidine Thiocyanate, 10mM Tris HCL pH 8.0 for 10min to denature proteins, rinsed in water and processed as for Western blotting using anti-PrP 6H4 (Prionics). Non-specific binding is blocked by incubation with 5% milk dissolved in PBS for 1hr. The membrane is then exposed to specific primary antibody anti-PrP 6H4, followed by HRP-conjugated secondary antibody cach diluted as appropriate in PBS, 0.3% Tween 20. Western blots are developed by ECL™ (Amersham) as directed according to the provider instructions.

The chemiluminescence signal from membranes is then analyzed directly using the Kodak Imagestation 440CF. The luminescence signal in each condition was normalized for possible differences in cell growth. Total protein content of a parallel lysate untreated with proteinase K is determined using the BCA assay (Biorad) and results are expressed as intensity/µg protein.

Of the 63 sub-clones analysed, 9 were found to be capable of replicating PrP^{Sc}, albeit with differing efficiencies (Figure 3). The remaining 54 sub-clones were resistant to infection. The most highly prion infection sensitive cell lines were selected for further study together with several prion infection resistant sub-clones with similar morphologies and doubling times. We have have selected two of these cell lines: #23, a prion infection resistant clone, and #60 a prion infection sensitive clone.

These two cell lines have been maintained in culture for over 1 year and have been infected with RML in many different occasions throughout this period: on every occasion sub-clone #60 was highly infectable whereas sub-clone #23 was totally resistant. Prion infection sensitive sub-clones could be maintained as a chronically infected cell culture by serial passaging at 1:15 or 1:20 dilution every 3 or 4 days respectively.

No evident morphological differences by microscopy were observed between the resistant or sensitive cells or between non-infected and infected cells.

To validate the clinical relevance of this cellular model of PrP relication, extracts of chronically infected N2a cells, or buffer alone, were injected into the hippocampus of normal mice by stereotactic injection. Injection of N2a extracts resulted in onset of clinical symptoms of scrapie after 140 days and premature death whereas mock injection had no effect on mouse physiology or life span. This indicates that the cell based model for prion replication using prion infection sensitive N2a cells generates infectious PrP scrapie, confirming that the conversion of PrP in cells is a good model for the process which occurs in vivo.

### c) Lipid rafts isolation:

Procedures for isolating lipid rafts based on resistance to solubilization in cold Triton X-100 followed by flotation on sucrose gradients have been described by numerous laboratories *(Simons et al., 2000; Hooper et al., 1999).* Lipid rafts from the two cell lines selected above are carried out as follows:

Subconfluent cultures of N2a cells are washed in PBS and collected by centrifugation 1000 x g for 5min. Typically 3 x 15cm dishes are pooled equivalent to approximately 8 x10⁷ cells. The cell pellet is re-suspended in 1ml ice cold raft buffer (1% Triton in PBS, containing 10µM copper sulphate and a cocktail of complete protease inhibitors (Boehringer)). Cells are disrupted by seven passages through a 22G needle followed by incubation for 30 min at 4°C with gentle agitation. 2 volumes 60% sucrose in PBS is added and the lysate is transferred to a SW41 centrifuge tube. The lysate is carefully overlaid with 7 ml 35% sucrose and 1ml 15% sucrose both in PBS and centrifuged 20hr at 35,000 RPM. The lipid rafts are recovered in the top 1ml of the gradient. Membranes are concentrated by addition of 10 volumes cold PBS and centrifugation at 100,000g for 2hr. Alternatively for 2D gel electrophoresis, proteins from the lipid raft fraction are recovered by precipitation in the presence of 5 vol acetone for 2hr at -80°C. Acetone precipitates are collected by centrifugation 14000g 20min and washed twice in 70% ethanol.

In both sensitive and resistant cells around 1-2% of protein in the total lysate is recovered in the bouyant raft fraction. As shown by Western blotting (Figure 4A) while PrP is barely detectable in the total cell extract, it is highly enriched in rafts leaving the sample layer totally depleted of PrP following centrifugation.

Prion infection sensitive clone #60 and the prion infection resistant clone #23 are compared by westerm blotting with anti-PrP (Figure 4B). Three different independent pairs of raft preparations each containing 5 µg total raft proteins are re-probed with anti-actin antibody which confirms the uniformity of PrP protein loading (Figure 4C).

The results indicate that the level of PrP in the lipid raft preparations from the two cell types is indistinguishable. Moreover the distribution between non glycosylated mono- and di-glycosylated isoforms as well as the segregation to the detergent resistant membrane fraction shown in Figure 4A is identical suggesting that none of these factors are likely to be responsible for the differing phenotypes.

PrP cDNA was amplified by RT-PCR from both cell lines as follows:
Total RNA of N2a cells is prepared using Trizol (Gibco) and the mouse PrP cDNA is reversed transcribed with Omniscript (Qiagen) using the protocol supplied by the manufacturer. The specific primer for cDNA synthesis is 5` TCAATTGAAAGAGCTACAGGTG 3'. The prion cDNA is amplified using standard PCR conditions in the presence of primers 5' ACCAGTCCAATTTAGGAGAGCC 3' (top strand) and 5' AGACCACGAGAATGCGAAGG 3' (bottom strand). The PCR product was completely sequenced in the automated ABI3700 using the reagents and the protocol supplied by the manufacturer.

These data revealed that PrP mRNA is wild type in both cases and that both carry a Methionine at position 129, which in humans is the site for a frequent polymorphism.

Therefore, the expression levels, glycosylation patterns, intracellular localisation and primary sequences of PrP^{C} in both cell types is indistinguishable and thus that other cellular factors are responsible for the differential response to PrP^{Sc}.

### d) In vitro cyclic amplification of protein misfolding (PMCA) in lipid rafts from prion infection sensitive cells:

Lipid rafts obtained at §c are isolated from prion infection sensitive sub-clones, #60 sub-clones, collected by centrifugation as described above and re-suspended in PMCA conversion buffer at a concentration of 2-2.5 mg/ml (PBS containing final concentration of 300mM NaCl, 0.5% Triton X100, 0.05% SDS).

A 10% extract of RML-infected mouse brain homogenate is added directly to the rafts preparation at a dilution of 1:100 based on protein content and aliquots of the mixture are either frozen immediately, incubated for 15hr at 37°C or subjected to 15 cycles of PMCA (5 x 0.1 second pulses of sonication followed by incubation at 37 °C for 1hr).

Aliquots of 20µl sample are then treated with 10 µg /ml Proteinase K for 1hr at 37°C. Lipids are removed by precipitating PK-resistant proteins with 5 vol acetone for 2hr at -80°C. Acetone precipitates are collected by centrifugation 14000g 20 min, washed twice in 70% ethanol analysed by Western blotting with 6H4 anti-PrP (Figure 5).

Compared to the mixture without PK treatment (lanes 1 and 5) all digested samples show a shift in molecular weight characteristic of the N-terminally truncated PK resistant form PrP₂₇₋₃₀. It should be noted that the 6H4 antibody also has low level cross reactivity with PK which migrates at 30kDa, close to the di-glycosylated form of PK-digested PrP. Analysis of the data with this in mind shows that the initial level of PK-resistant PrP derived from the diluted brain extract, which is present in the non-amplified mixtures, is barely detectable under these conditions (lanes 2).

A slight increase in signal is seen when the prion infection sensitive (#60) DRM is incubated at 37 °C for 15 hr (lane 3 from #60), however the most dramatic increase in PK-resistant PrP is seen when this sample is subjected to 15 cycles of PMCA (lane 4 from #60). This indicates that all factors required for conversion of PrP^{c} to PrP^{Sc} are resident in the lipid rafts from the prion infection sensitive N2a cells. Interestingly, in the parallel analysis in which the DRM from the prion infection resistant cell line #23 was used, no amplification in vitro was observed (lane 4 from #23) indicating that the capacity of the lipid rafts to convert the prion protein in vitro reflects the activity observed in the intact cells.

### e) Effect of antibody raised against apolipoprotein B on prion replication by prion infection sensitive N2a cells:

Chronically infected sensitive cells were cultured in 24-well dishes in the presence of a goat polyclonal antibody raised against human Apo B (Chemicon) at increasing concentrations from 0 to 2 mg/ml in DMEM Gibco # 41966-029, containing 1 x B27 supplements (Gibco #17504-044) and standard antibiotic (penicillin and streptomycin)

A parallel series of cultures was incubated in the presence of the same concentration range of total IgG from a naïve goat. The results show that concentrations of anti-hApoB antibody above 0.5mg/ml result in progressive inhibition of PrP replication as revealed by quantitative dot blotting (Figure 6).

These data show the role of Apolipoprotein B in the prion conversion.

### EXAMPLE 3: Proteomics analysis of lipid rafts of prion infection resistant and sensitive cells:

Since the two cell preparations are indistinguishable in terms of their PrP content a more complete protein comparison using 2D gel electrophoresis was performed to show differences in other proteins that might underline the difference in conversion activity between the two sub-clones.

2D gel preparations are prepared as follows:
Acetone precipitated proteins (see §c) are re-suspended in 20µl 1% SDS, 0.23% DTT and heated to 95°C for 5min. After the preparation is cooled to room temperature, 25µl of a solution (9M urea, 4% CHAPS, 65mM DTT, 35mM Tris base) is added.
   Fifteen minutes later, 85µl of a solution containing 7M urea, 2M thiourea, 4% CHAPS, 100mM DTT is further added to the mixture. After a further 15 min, non-solubilized material is removed by centrifugation at 14000 RPM during 5min and the supernatant is applied directly to a 7cm IPG strip and left to re-hydrate overnight. For IEF the voltage is progressively increased from 300V to 3.5kV and electrophoresed for a total of 20kVh. Proteins are resolved in the second dimension using single well 4-12% gradient gels (Novex) and stained using the silver express kit (Invitogen) according to the instructions supplied.

Analysis by 2D gels reveals the fraction of protein that is recovered in the lipid rafts (approximately 1-2% protein in the N2a cell lysate) as a reproducible subset of total cell proteins in which several hundred species can be visualized following silver staining (Figure 7A and B).

The 2D patterns are compared between preparations isolated from the prion infection sensitive and resistant cells. The analysis is focused on several proteins identified in the basic range of the gel, which are more abundant in DRMs from prion infection resistant cells (arrows in Figure 7C and D).
Following preparative scale electrophoresis, the two proteins indicated by arrows are excised and processed for MS sequencing. From both proteins an identical tryptic peptide is found with a monoisotopic mass of 1234.6. The N-terminal sequence of this tryptic peptide is: ENFAGEATLQR (SEQ ID NO: 3). All amino acids in the peptide are identified in the MS/MS spectrum of doubly charged precursor ion at m/z 618.30 And through its Mascot analysis.

Database searching identified this protein unambiguously as Apolipoprotein B (Apo B). Since the molecular weight of full length Apo B is in excess of 500 kDa while these two spots migrate with estimated molecular weights of 40kDa and 30kDa, we presume that the latter are fragments generated either in the cell or during sample preparation. The sequence corresponds to amino acids 3548-3558 of the human Apo B protein, which is present only in ApoB-100 and not in the truncated ApoB-48 form.

These data suggest that fragments of a molecular weight of or about 30 to 40 kDa comprising the sequence of SEQ ID NO: 3 may have a role in the prion conversion pathway.

### EXAMPLE 4: Binding and internalisation of fluorescent LDL receptor by resistant and sensitive cells:

N2a subclones #23 (prion infection sensitive) and #60 (prion infection resistant) were cultured in 24 well plates in standard DMEM medium containing 10% FCS for 2 days then transferred to the same medium (300 µl) containing 1% FCS for 1hr. To visualize cell surface binding, plates were placed on ice to inhibit endocytosis and 3µl fluorescent DiI-LDL (Molecular Probes) was added for 30 min. LDL-binding was visualized by fluorescence microscopy. To study LDL uptake by each of the sub-clones, cells were incubated at 37 C with 3µl DiI-LDL for 2h prior to microscopic examination.
Control cultures were incubated in parallel with DiI-coupled *acetylated* LDL which does not bind the LDL receptor or with Hoechst to visualize cell nuclei.

The binding or uptake of fluorescent DiI-LDL is similar for prion infection resistant and prion infection sensitive cells, suggesting that the level of the LDL receptor between these two cell types is similar.

### References

Aizawa et al., Brain R. 768 (1-2), 208-14, 1997;
Baron et aL, The EMBO Journal, 21, 5, 1031-1040,2002;
Baumann et al., Biochem J., 349, 77-8, 2000;
Bruce et aL, Nature, 389, 498-501, 1997;
Bueler et al., Cell 73,1339-1347,1993;
Chabry et al., J. BioL Chem. 273, 13203-13207, 1998;
Choe et al., Electrophoresis, 23, 2242-2246, 2002;
Choi et al., J. Lip. Res., 38(1)77-85,1997;
Clavey et al., Annales d'Endocrinologie, 52, 459-463, 1991;
Cohen et al., Ann. Rev. Biochem. 67, 793-819, 1998;
Dietrich et al., Journal of virology, 65(9), 4759-476,1991;
Enari et al, Proc. Natl. Acad. Sci. USA 98, 9295-9299, 2001;
Fantini et al., Expert Reviews in Molecular Medicine, Dec 20, 1-22, 2002;
Golaz et aL, Electrophoresis, 16, 1184-118, 1995;
Hooper et al., Mol. Memb. BioL 16,145-156,1999;
Lehninger et al., Principles of Biochemistry, 2nd Ed. New York: Worth Publishers, 1993;
Lingappa et al, Membrane proteins, Kyushu Univ. Press Japon, 1997, pp 93-100, XP001183818 & Internat. Symposium ISSN:0003-4266
Lucassen et al., Biochemistry, 42, 4127-4135, 2003;
Pan et al., Prey. Natl. Acad. Sci. (USA) 90,10962-10966,1993;
Prusiner, Science 252, 1515-1522, 1991;
Prusiner, Proc. Natl. Acad. Sci. USA 95,13363-13383,1998;
Roos et al., Brain 96,1-20, 1973;
Saborio et al., Biochem. Biophys. Res. Commun. 258, 470-475, 1999;
Saborio et al., Nature 411, 810-813, 2001;
Schulz et al., American Journal of Pathology, 156(1), 51-56, 2000;
Segrest et al., Journal of Lipid Research, 42, 1346-1367, 2001;
Simons ct al, Molecular Cell Biology 1, p 31-41, 2000;
Scott et al., Proc. Natl. Acad. Sci. USA 96, 15137-15142,1999;
Soto et aL, Trends Mol. Med. 7, 109-714, 2001;
Taraboulos at al., The Journal of Cell Biology, 129 (1), 121-132,1995;
Telling et al., Proc. Natl. Acad. Sci. USA 91, 9936-9940, 1994;
Tsui-Pierchala et aL, Trends Neurosci. 25,412-417,2002;
Wang et al., Aeterioscler. Thromb. Vas. Biol., 20(5), 1301-8,2000;
Will et al., Lancet 347, 925, 1996;
Yamada et al, Ann Clin. Lab. Sci. 27(4), 77-85,1997;
US 5,134,121;
US 5,276,059;
US 5,948,763;
US 6,022,683;
US 6,197,972;
US 6,355,610;
US 6,552,922;
US 20020128175;
US 20020155426;
WO 97/14437;
WO 99/15159;
WO 0168710;
WO 0204954;
WO 02065133;
WO 03002533;
WO 03005037;
WO 03045921;
WO 2004043403.

### SEQUENCE LISTING

<110> APPLIED RESEARCH SYSTEMS ARS HOLDING N.V.
<120> USE OF PRION CONVERSION MODULATING AGENTS
<130> WO/845
<160> 3
<170> PatentIn version 3.1
<210> 1
   <211> 4563
   <212> PRT
   <213> Homo sapiens
<400> 1
<210> 2
   <211> 317
   <212> PRT
   <213> Homo sapiens
<400> 2
<210> 3
   <211> 11
   <212> PRT
   <213> synthetic construct
<400> 3
   Glu Asn Phe Ala Gly Glu Ala Thr Leu Gln Arg
   1               5                   10

## Claims

1. Use of Apolipoprotein B, in an assay for the detection of the formation of PrP^{sc} in a sample.

2. Use of Apolipoprotein B in a screening assay for identifying compounds that modulate the conversion of PrP^{c} into PrP^{sc}.

3. Use according to claims 1 or 2 wherein Apolipoprotein B forms a complex with the LDL receptor.

4. Use according to any of the preceding claims wherein the assay is a Protein Misfolding Cyclic Amplification (PMCA) assay.

5. Use according to any of the preceding claims wherein the assay is a Protein Misfolding Cyclic Amplification (PMCA) assay using normal brain homogenate as a source of normal PrP^{C} and substrate.

6. Use according to claims 1 to 5 wherein the assay is a Protein Misfolding Cyclic Amplification (PMCA) assay using lipid rafts from infection sensitive neuroblasma cell line N2a as a source of normal PrP^{C} and substrate.

7. Use of an antibody raised against Apolipoprotein B for the preparation of a pharmaceutical composition for the treatment of a prion disease.

8. Use according to claim 7 wherein the prion disease is bovine spongiform encephalopathy (BSE).

9. Use according to claim 7 wherein the prion disease is a Creutzfeld-Jacob Disease (CJD).

10. A method for the diagnosis or detection of a prion disease within a subject suspected of suffering from such a disease which comprises (i) contacting a sample from said subject with Apolipoprotein B; (ii) contacting the mixture obtained in step (i) with PrP^{C} or PrP^{C} containing mixtures; and (iii) determining the presence and/or amount of PrP^{Sc} in said sample.

11. A method of determining a marker that predisposes a subject to a prion disease, comprising (i) measuring the level of Apolipoprotein B in a sample; and (ii) correlating said level of protein obtained in said measuring step with the occurrence of a prion disease.

12. A method according to any one of claims 10 to 11 wherein the prion disease is bovine spongiform encephalopathy (BSE).

13. A method according to any one of claims 10 to 11 wherein the prion disease is a Creutzfeld-Jacob disease.

14. A method for the detection of Pr^{Sc} within a sample, which assay comprises (i) contacting said sample with Apolipoprotein B; (ii) contacting sample obtained in (i) with PrP^{C} or PrP^{C} containing mixtures; and (iii) determining the presence and/or amount of PrP^{Sc} in said sample.

15. A method for identifying, in a sample, a compound which modulates the transition of PrP^{C} into PrP^{Sc} comprising: (i) contacting said sample with Apolipoprotein B; (a) in the presence of said modulatory compound and (b) in the absence of said compound; (ii) contacting the mixtures obtained in step (i) a and (i) b with PrP^{C} or PrP^{C} containing mixtures; and (iii) determining the amount of PrP^{Sc} (a) in the presence of said modulatory compound and (b) in the absence of said modulatory compound.

16. An assay for the detection of PrP^{Sc} in a sample, which assay comprises (i) contacting said sample with Apolipoprotein B (ii) contacting the mixture obtained in step (i) with PrP^{C} or PrP^{C} containing mixtures; (iii) determining the presence and/or amount of PrP^{Sc} in said sample.

17. A screening assay for identifying, a compound which modulates the transition of PrP^{C} into PrP^{Sc} comprising: (i) contacting said sample with Apolipoprotein B; (a) in the presence of said modulatory compound and (b) in the absence of said modulatory compound; (ii) contacting the mixtures obtained in step (i) a and (i) b with PrP^{C} or PrP^{C} containing mixtures; and (iii) determining the amount of PrP^{Sc} (a) in the presence of said compound and (b) in the absence of said modulatory compound.

## Patentansprüche

1. Verwendung von Apolipoprotein B in einem Assay zum Nachweisen der Bildung von PrP^{SC} in einer Probe.

2. Verwendung von Apolipoprotein B in einem Screening-Assay zum Identifizieren von Verbindungen, die die Umwandlung von PrP^{C} in Pr^{SC} modulieren.

3. Verwendung nach den Ansprüchen 1 oder 2, wobei Apolipoprotein B einen Komplex mit dem LDL-Rezeptor bildet.

4. Verwendung nach einem der vorhergehenden Ansprüche, wobei der Assay ein Protein Misfolding Cyclic Amplification (PMCA) Assay ist.

5. Verwendung nach einem der vorhergehenden Ansprüche, wobei der Assay ein Protein Misfolding Cyclic Amplification (PMCA) Assay ist, bei dem normales Gehimhomogenat als Quelle für normales PrP^{C} und Substrat verwendet wird.

6. Verwendung nach den Ansprüchen 1 bis 5, wobei der Assay ein Protein Misfolding Cyclic Amplification (PMCA) Assay ist, bei dem Lipid-Rafts aus der infektionsempfindlichen Neuroblastom-Zelllinie N2a als Quelle für normales PrP^{C} und Substrat verwendet wird.

7. Verwendung eines Antikörpers, der gegen Apolipoprotein B zur Herstellung einer pharmazeutischen Zusammensetzung zur Behandlung einer Prionerkrankung erzeugt wird.

8. Verwendung nach Anspruch 7, wobei die Prionerkrankung bovine spongiforme Enzephalopathie (BSE) ist.

9. Verwendung nach Anspruch 7, wobei die Prionerktankung eine Creutzfeld-Jacob-Erkrankung (CJD) ist.

10. Verfahren zur Diagnose oder zum Nachweis einer Prionerkrankung in einem Individuum, bei dem der Verdacht besteht, dass es an einer solchen Krankheit leidet, umfassend (i) das Zusammenbringen einer Probe aus dem Individuum mit Apolipoprotein B; (ii) das Zusammenbringen des in Schritt (i) erhaltenen Gemischs mit PrP^{C} oder PrP^{C}-enthaltenden Gemischen; und (iii) das Bestimmen der Anwesenheit und/oder der Menge von Prp^{Sc} in der Probe.

11. Verfahren zur Bestimmung eines Markers, der bei einem Individuum eine Veranlagung zu einer Prionerkrankung schafft, umfassend (i) das Messen der Menge von Apolipoprotein B in einer Probe; und (ii) das Korrelieren der in dem Messschritt erhaltenen Proteinmenge mit dem Auftreten einer Prionerkrankung.

12. Verfahren nach einem der Ansprüche 10 bis 11, wobei die Prionerkrankung bovine spongiforme Enzephalopathie (BSE) ist.

13. Verfahren nach einem der Ansprüche 10 bis 11, wobei die Prionerkrankung eine Creutzfeld-Jacob-Krankheit ist.

14. Verfahren zum Nachweisen von PrP^{Sc} in einer Probe, wobei der Assay umfasst (i) das Zusammenbringen der Probe mit Apolipoprotein B; (ii) das Zusammenbringen der in (i) erhaltenen Probe mit PrP^{C} oder PrP^{C}-enthaltenden Gemischen; und (iii) das Bestimmen der Anwesenheit und/oder der Menge von PrP^{Sc} in der Probe.

15. Verfahren zum Identifizieren einer Verbindung, die den Übergang von PrP^{C} in PrP^{Sc} moduliert, in einer Probe, umfassend: (i) das Zusammenbringen der Probe mit Apolipoprotein B, (a) in der Anwesenheit der modulatorischen Verbindung und (b) in der Abwesenheit der Verbindung; (ii) das Zusammenbringen der in Schritt (i) a und (i) b erhaltenen Gemische mit PrP^{C} oder PrP^{C}-enthaltenden Gemischen; und (iii) das Bestimmen der Menge von PrP^{Sc} (a) in der Anwesenheit der modulatorischen Verbindung und (b) in der Abwesenheit der modulatorischen Verbindung.

16. Assay zum Nachweisen von PrP^{Sc} in einer Probe, wobei der Assay umfasst (i) das Zusammenbringen der Probe mit Apolipoprotein B; (ii) das Zusammenbringen des in Schritt (i) erhaltenen Gemischs mit PrP^{C} oder PrP^{C}-enthaltenden Gemischen; (iii) das Bestimmen der Anwesenheit und/oder der Menge von PrP^{Sc} in der Probe.

17. Screening Assay zum Identifizieren einer Verbindung, die den Übergang von PrP^{C} in PrP^{Sc} moduliert, umfassend: (i) das Zusammenbringen der Probe mit Apolipoprotein B; (a) in der Anwesenheit der modulatorischen Verbindung und (b) in der Abwesenheit der modulatorischen Verbindung; (ii) das Zusammenbringen der in Schritt (i) a und (i) b erhaltenen Gemische mit PrP^{C} oder PrP^{C}-enthaltenden Gemischen; und (iii) das Bestimmen der Menge von PrP^{Sc} (a) in der Anwesenheit der Verbindung und (b) in der Abwesenheit der modulatorischen Verbindung.

## Revendications

1. Utilisation d'apolipoprotéine B au niveau d'un test pour la détection de la formation de PrP^{Sc} dans unéchantillon.

2. Utilisation d'apolipoprotéine B au niveau d'un test de filtrage pour identifier des composés qui modulent la conversion de PrP^{C} en PrP^{Sc}.

3. Utilisation selon la revendication 1 ou la revendication 2, dans laquelle l'apolipoprotéine B forme un complexe avec le récepteur LDL.

4. Utilisation selon l'une quelconque des revendications précédentes, dans laquelle le test est un test d'amplification cyclique de défaut de repliement de protéine (PMCA).

5. Utilisation selon l'une quelconque des revendications précédentes, dans laquelle le test est un test d'amplification cyclique de défaut de repliement de protéine (PMCA) qui utilise un broyat de cerveau normal en tant que source de PrP^{C} normal et que substrat.

6. Utilisation selon l'une quelconque des revendications 1 à 5, dans laquelle le test est un test d'amplification cyclique de défaut de repliement de protéine (PMCA) qui utilise des rafts de lipides issus d'une ligne de cellules de neuroblasme sensible à l'infection N2a en tant que source de PrP^{C}normal et que substrat.

7. Utilisation d'un anticorps envisagé à l'encontre d'apolipoprotéine B pour la préparation d'une composition pharmaceutique pour le traitement d'une maladie par prions.

8. Utilisation selon la revendication 7, dans laquelle la maladie par prions est l'enoéphalopathie spongiforme bovine (BSE).

9. Utilisation selon la revendication 7, dans laquelle la maladie par prions est la maladie de Creutzfeld-Jacob (CJD).

10. Procédé pour le diagnostic ou la détection d'une maladie par prions chez un sujet suspecté de souffrir de cette maladie, lequel comprend : (i) la mise en contact d'un échantillon issu dudit sujet avec de l'apolipoprotéine B ; (ii) la mise en contact du mélange obtenu au niveau de l'étape (i) avec PrP^{C} ou des mélanges contenant PrP^{C} ; et (iii) la détermination de la présence et/ou de la quantité de PrP^{Sc} dans leditéchantillon.

11. Procédé de détermination d'un marqueur qui prédispose un sujet à une maladie par prions, comprenant : (i) la mesure d'un niveau d'apolipoprotéine B dans un échantillon ; et (ii) la corrélation dudit niveau de protéine obtenu lors de laditeétape de mesure avec la survenue d'une maladie par prions.

12. Procédé selon l'une quelconque des revendications 10 et 11, dans lequel la maladie par prions est l'encéphalopathie spongiforme bovine (BSE).

13. Procédé selon l'une quelconque des revendications 10 et 11, dans lequel la maladie par prions est la maladie de Creutzfeld-Jacob (CJD).

14. Procédé pour la détection de PrP^{Sc} dans un échantillon, lequel comprend : (i) la mise en contact dudit échantillon avec de l'apolipoprotéine B ; (ii) la mise en contact de l'échantillon obtenu en (i) avec PrP^{C} ou des mélanges contenant PrP^{C} ; et (iii) la détermination de la présence et/ou de la quantité de PrP^{Sc} dans leditéchantillon.

15. Procédé pour identifier, dans un échantillon, un composé qui module la transition de PrP^{C} en PrP^{Sc} comprenant : (i) la mise en contact dudit échantillon avec de l'apolipoprotéine B (a) en présence dudit composé modulatoire et (b) en l'absence dudit composé ; (ii) la mise en contact des mélanges obtenus au niveau de l'étape (i) (a) et (i) (b) avec PrP^{C} ou des mélanges contenant PrP^{C}; et (iii) la détermination de la quantité de PrP^{Sc} (a) en présence dudit composé modulatoire et (b) en l'absence dudit composé modulatoire.

16. Test pour la détection de PrP^{Sc} dans un échantillon, comprenant : (i) la mise en contact dudit échantillon avec de l'apolipoprotéine B ; (ii) la mise en contact du mélange obtenu au niveau de l'étape (1) avec PrP^{C} ou des mélanges contenant PrP^{C} ; (iii) la détermination de la présence et/ou de la quantité de PrP^{Sc} dans leditéchantillon.

17. Test de filtrage pour identifier un composé qui module la transition de PrP^{C} en PrP^{Sc} comprenant : (i) la mise en contact duditéchantillon avec de l'apolipoprotéine B (a) en présence dudit composé modulatoire et (b) en l'absence dudit composé modulatoire ; (ii) la mise en contact des mélanges obtenus au niveau de l'étape (i) (a) et (i) (b) avec PrP^{C} ou des mélanges contenant PrP^{C} ; et (iii) la extermination de la quantité de PrP^{Sc} (a) en présence dudit composé et (b) en l'absence dudit composé modulatoire.
